# EUROPEAN PATENT APPLICATION

(11) **EP 4 327 821 A1**
(43) Date of publication of application: **28.02.2024**
(21) Application number: 22811609.1
(22) Date of filing: 24.05.2022
(51) Int. Cl.: A61K 39/39, A61K 9/08, A61K 39/395, A61K 38/17, A61K 31/704, A61K 45/06, A61P 35/00, A61K 39/00

(54) **IMMUNO-ONCOLOGY THERAPEUTIC COMPOSITION USING ADJUVANT INCLUDING LIPOPEPTIDES AND POLY (I:C)**

(30) Priority: 24.05.2021 KR 20210065980
(71) Applicant: Cha Vaccine Research Institute Co., Ltd, Seongnam-si, Gyeonggi-do 13230 (KR)
(72) Inventor: YUM, Jung Sun, Seongnam-si Gyeonggi-do 13230 (KR); CHO, Jungki, Seongnam-si Gyeonggi-do 13230 (KR); AHN, Byung Cheol, Seongnam-si Gyeonggi-do 13230 (KR); JEONG, Sookyung, Seongnam-si Gyeonggi-do 13230 (KR); HEO, Yoonki, Seongnam-si Gyeonggi-do 13230 (KR); JEON, Hong Jae, Seongnam-si Gyeonggi-do 13528 (KR); KIM, Chan, Yongin-si Gyeonggi-do 16903 (KR)
(74) Representative: EP&C
(86) International application number: PCT/KR2022/007348
(87) International publication number: WO 2022/250416

(57) **Abstract**

An immuno-oncology therapeutic composition containing, as an active ingredient, an adjuvant including lipopeptides and poly (I:C) provided in one aspect of the present invention can induce a large therapeutic effect on a variety of carcinomas, and can be effectively used for anticancer therapy by significantly enhancing anticancer effects through combined administration with conventional anticancer drugs, such as chemical anticancer drugs, anticancer vaccines, and immune checkpoint inhibitors, having different mechanisms.

## Description

### BACKGROUND OF THE INVENTION

### 1. Field of the Invention

The present invention relates to an immuno-oncology therapeutic composition comprising an adjuvant including lipopeptides and poly (I:C) as an active ingredient.

The present invention also relates to an immuno-oncology therapeutic composition having increased anticancer immune activity by co-administering an adjuvant including lipopeptides and poly (I:C) with an immune checkpoint inhibitor or other TLR ligands.

### 2. Description of the Related Art

TLR (toll-like receptor) is a cell receptor that induces an innate immune response. It recognizes a pathogen-associated molecular pattern (PAMP) derived from initial pathogens and activates an innate immune defense mechanism through a signaling pathway, thereby protecting against pathogen invasion from the beginning. TLR also plays an important immune role by activating dendritic cells and activating adaptive immune responses such as T and B cells.

TLR, which is known to be expressed in most immune cells, is also known to be expressed in various cancer cells (Non-patent reference 1, Cancer Microenvironment (2009) 2 (Suppl 1) :S205-S214, Cancer Cells Expressing Toll-like Receptors and the Tumor Microenvironment).

**[Table 1]**

| **Type of cancer** | **TLR** |
|---|---|
| Gastric cancer | TLR2, TLR4, TLR5, TLR9 |
| Colorectal cancer | TLR2, TLR3, TLR4, TLR5, TLR9 |
| Ovarian cancer | TLR2, TLR3, TLR4, TLR5 |
| Cervical cancer | TLR3, TLR4, TLR5, TLR9 |
| Lung cancer | TLR2, TLR3, TLR4, TLR9 |
| Prostate cancer | TLR4, TLR9 |
| Melanomas | TLR2, TLR3, TLR4 |
| Brain cancer | TLR2, TLR4 |
| Breast cancer | TLR2, TLR3, TLR4, TLR9 |
| Hepatocellular carcinoma | TLR2, TLR3, TLR4, TLR6, TLR9 |
| Laryngeal cancer | TLR2, TLR3, TLR4 |

TLR expressed in cancer cells can increase the immune response in the tumor microenvironment by inducing the infiltration of immune cells into cancer cells through the secretion of cytokines and chemokines in cancer cells.

In addition, it has been reported that TLR stimulating substances (TLR ligands) induce cell death when treated directly to cancer cells. TLR ligands induce apoptosis of cancer cells by activating the caspase pathway within cancer cells, causing cell death, and type I IFN activated by TLR ligands further promotes apoptosis through the activation of immune cells.

When cancer cells are killed by TLR ligands, they release intrinsic proteins and immunogenic cell death markers, which activate dendritic cells and then cancer cell-specific NK cells and T cells kill cancer cells.

In addition, when cancer cells are killed by TLR ligands, the exposure of cancer antigens derived from cancer cells and the secretion of DAMP (damage associated molecular pattern) are promoted. The exposed cancer antigens can induce a cancer antigen-specific immune response by the adjuvant effect of TLR ligands. Therefore, it is expected that TLR ligands can be developed as immuno-oncology drugs by inducing cancer cell death and cancer antigen-specific immune responses.

Immune checkpoint proteins are cell membrane proteins that inhibit the differentiation, proliferation, and activity of immune cells, but they are expressed not only in immune cells but also in cancer cells. Immune checkpoint proteins such as PD-L1 expressed in cancer cells are known to play an important role in protecting cancer cells from T-cell immune attack by inactivating cancer-specific T cells, thereby inducing the immune evasion mechanism of cancer. By inhibiting the function of these immune checkpoint proteins, the immune checkpoint inhibitors help immuno-oncology drugs eliminate cancer cells.

Immune checkpoint inhibitors are known to have a high therapeutic effect in about 30% of patients when treated alone, but have no therapeutic effect in the remaining patients. It is known that the reason for the limited effectiveness of immune checkpoint inhibitors is the low response rate and resistance to immune checkpoint inhibitors.

The low response rate of immune checkpoint inhibitors is due to the different expression levels of immune checkpoint proteins in different cancers, with lower response rates in low-expression cancers. In addition, even if the evasion mechanism is suppressed through immune checkpoint inhibitors, the cancer-specific immune response that can kill cancer cells is not induced due to low exposure to cancer antigens, resulting in a low response rate. Therefore, the limitations of immune checkpoint inhibitors can be overcome only through combined use of methods that can increase exposure of cancer antigens and increase cancer-specific immune responses.

Resistance to immune checkpoint inhibitors occurs because tumor-specific immune cells are not able to enter the cancer tissue, forming an early hypoimmunogenic environment without infiltration of immune cells within the cancer tissue, resulting in resistance to immunotherapy.

In order to overcome the low response rate and resistance of the immune checkpoint inhibitors, it is necessary to convert the low immunogenic tumor environment into the high immunogenic tumor environment, which can be solved by increasing immunogenicity using TLR ligands.

The present inventors have developed a vaccine composition comprising a lipopeptide, which is a TLR2 ligand, and a poly(I:C), which is a TLR3 ligand, in previous studies, and have confirmed that the vaccine composition induces a strong immune response (Korean Patent No. 10-0900837, Korean Patent No. 10-1501583), and have named the vaccine composition as L-pampo.

In this invention, the present inventors discovered that L-Pampo induces a strong immune anti-cancer response and completed the present invention by confirming that the L-Pampo administered to tumor cells inhibits cancer cell growth, induces apoptosis, and inhibits metastasis by using the anticancer effect of changing the tumor microenvironment and inducing an immune response specific to cancer antigens, and that the combination of L-Pampo with other TLR ligands, saponin or immune checkpoint inhibitors overcomes the immune evasion of cancer cells and dramatically improves the anti-cancer efficacy.

### SUMMARY OF THE INVENTION

It is an object of the present invention to provide a pharmaceutical composition comprising an adjuvant including lipopeptides and poly (I:C) as an active ingredient for the prevention or treatment of cancer.

It is another object of the present invention to provide a method of generating an immune response against cancer in a subject, comprising a step of administering the pharmaceutical composition comprising an adjuvant including lipopeptides and poly (I:C) as an active ingredient for the prevention or treatment of cancer to a subject except humans.

It is another object of the present invention to provide a combination formulation comprising an adjuvant including lipopeptides and poly (I:C), and an immune checkpoint inhibitor as active ingredients for the prevention or treatment of cancer.

It is another object of the present invention to provide a combination formulation comprising an adjuvant including lipopeptides and poly (I:C), and a TLR ligand as active ingredients for the prevention or treatment of cancer.

It is another object of the present invention to provide a combination formulation comprising an adjuvant including lipopeptides and poly (I:C), and saponin as active ingredients for the prevention or treatment of cancer.

To achieve the above objects, in an aspect of the present invention, the present invention provides a pharmaceutical composition comprising an adjuvant including lipopeptides and poly (I:C) as an active ingredient for the prevention or treatment of cancer.

In another aspect of the present invention, the present invention provides a method of generating an immune response against cancer in a subject, comprising a step of administering the pharmaceutical composition comprising an adjuvant including lipopeptides and poly (I:C) as an active ingredient for the prevention or treatment of cancer to a subject except humans.

In another aspect of the present invention, the present invention provides a combination formulation comprising an adjuvant including lipopeptides and poly (I:C), and an immune checkpoint inhibitor as active ingredients for the prevention or treatment of cancer.

In another aspect of the present invention, the present invention provides a combination formulation comprising an adjuvant including lipopeptides and poly (I:C), and a second component including a TLR (toll-like receptor) ligand as active ingredients for the prevention or treatment of cancer.

In another aspect of the present invention, the present invention provides a combination formulation comprising an adjuvant including lipopeptides and poly (I:C), and saponin as active ingredients for the prevention or treatment of cancer.

### ADVANTAGEOUS EFFECT

An immuno-oncology therapeutic composition containing, as an active ingredient, an adjuvant including lipopeptides and poly (I:C) provided in one aspect of the present invention can induce a large therapeutic effect on a variety of carcinomas, and can be effectively used for anticancer therapy by significantly enhancing anticancer effects through combined administration with conventional anticancer drugs, such as chemical anticancer drugs, anticancer vaccines, and immune checkpoint inhibitors, having different mechanisms.

### BRIEF DESCRIPTION OF THE DRAWINGS

Figure 1 is a graph confirming the caspase-3 activity of an immuno-oncology drug in a mouse colon cancer cell line.
Figure 2 is a graph confirming the tumor suppressive efficacy of an immuno-oncology drug in a melanoma mouse model.
Figure 3 is a graph confirming the tumor suppressive efficacy of an immuno-oncology drug in a colon cancer mouse model.
Figure 4 is a set of photographs showing the results of analyzing the effect of an immuno-oncology drug on immune cell infiltration in the tumor using immunofluorescence staining in a colon cancer mouse model.
Figure 5 is a set of graphs showing the results of analyzing the effect of an immuno-oncology drug on immune cell infiltration in the tumor using flow cytometry in a colon cancer mouse model.
Figure 6 is a set of photographs and a graph showing the results of analyzing the induction of tumor-specific cellular immune responses by an immuno-oncology drug using ELISPOT assay in a colon cancer mouse model.
Figure 7 is a set of a diagram and graphs showing the results of analyzing the abscopal efficacy of an immuno-oncology drug in a colon cancer mouse model.
Figure 8 is a graph confirming the tumor suppressing efficacy of an immuno-oncology drug in a bladder cancer mouse model.
Figure 9 is a graph confirming the tumor suppressing efficacy of an immuno-oncology drug in a pancreatic cancer mouse model.
Figure 10 is a set of graphs confirming the efficacy of an immuno-oncology drug in combination with an immune checkpoint inhibitor in a colon cancer mouse model.
Figure 11a is a graph confirming the efficacy (tumor size) of an immuno-oncology drug in combination with other TLR ligands or saponin in a colon cancer mouse model.
Figure 11b is a graph confirming the efficacy (tumor weight) of an immuno-oncology drug in combination with other TLR ligands or saponin in a colon cancer mouse model.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In an aspect of the present invention, the present invention provides a pharmaceutical composition comprising an adjuvant including lipopeptides and poly (I:C) as an active ingredient for the prevention or treatment of cancer.

The lipopeptide is a synthetic analogue of lipopeptides derived from bacteria and mycoplasma and was first synthesized by J. Metzger et al. (Metzger, J. et al., 1991, Synthesis of novel immunologically active tripalmitoyl-S-glycerylcysteinyllipopeptides as useful intermediates for immunogen preparations. Int. J. Peptide Protein Res. 37: 46-57). The molecular structure of the compound represented by the following formula (1) is N-palmitoyl-S-[2,3-bis(palmitoyloxy)-(2RS)-propyl]-[R]-cystein-SKKKK (pam3Cys-SKKKK), and various other analogs have been synthesized.

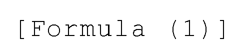

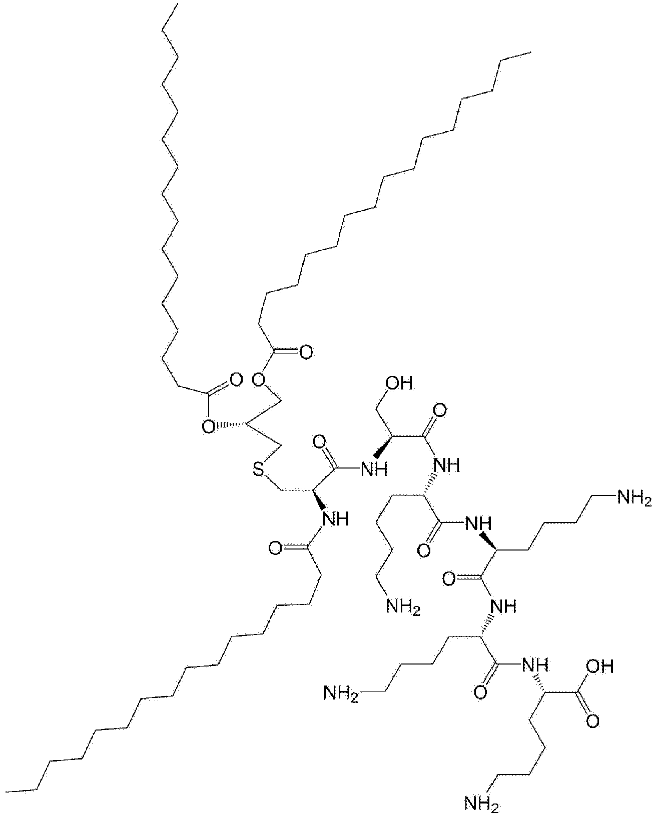

According to H. Schild et al., when Pam3Cys-Ser-Ser was combined with an influenza virus T cell epitope and administered to mice, virus-specific cytotoxic T lymphocytes (CTL) were induced. In general, lipopeptides are known as ligands for TLR2. The use of such lipopeptides is not limited to Pam3Cys-SKKKK, and lipopeptides may be composed of fatty acids and several amino acids bound to glycerol molecules. Specific examples include PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, Dhc-SKKKK, etc. The number of fatty acids in a molecule can be one or more. The number of amino acids in a lipopeptide can be one or more. In addition, fatty acids and amino acids can be chemically modified. Furthermore, lipopeptides can be lipoproteins, either as part of a molecule or as a whole molecule, derived from Gram-positive or Gram-negative bacteria or mycoplasma.

In addition, poly(I:C) has been used as a potent derivative of type 1 interferon in *in vitro* and *in vivo* studies. Moreover, poly(I:C) is known to stably and maturely form dendritic cells, the most potent antigen-presenting cells in mammals (Rous, R. et al 2004. poly(I:C) used for human dendritic cell maturation preserves their ability to secondarily secrete bioactive 11-12, International Immunol. 16: 767-773). According to these previous reports, poly(I:C) is a strong IL-12 inducer, and IL-12 is an important cytokine that promotes the development of Th1 and induces cellular immune responses and the formation of IgG2a or IgG2b. In addition, poly(I:C) is known to have strong adjuvant activity against peptide antigens (Cui, Z. and F. Qui. 2005. Synthetic double stranded RNA poly I:C as a potent peptide vaccine adjuvant: Therapeutic activity against human cervical cancer in a rodent model. Cancer Immunol. Immunotherapy 16: 1-13). Poly(I:C) is known as a representative TLR3 ligand. The length of poly(I:C) may range from 50 to 5,000 bp, preferably from 50 to 2,000 bp, and more preferably from 100 to 500 bp, but not always limited thereto.

The lipopeptide and poly(I:C) can be included in the pharmaceutical composition at a weight ratio of 0.1 to 10 : 1, 1.25 to 2 : 1, 1.25 to 1.5 : 1, or 1.25 : 1, but is not particularly limited thereto, and can be adjusted to an appropriate level based on the patient's condition. Further, the pharmaceutical composition can be an aqueous solution formulation.

The pharmaceutical composition can further include at least one selected from the group consisting of pharmaceutically acceptable carriers, diluents, and adjuvants. For example, the pharmaceutical composition can include a pharmaceutically acceptable carrier, and can be formulated for human or veterinary use and administered through various routes. The route of administration can be oral, intraperitoneal, intravenous, intramuscular, subcutaneous, intradermal, etc. Preferably, it is formulated and administered as an injection. Injectables can be prepared using aqueous solvents such as physiological saline and Ringer's solution, and non-aqueous solvents such as vegetable oils, higher fatty acid esters (e.g., ethyl oleate, etc.), alcohols (e.g., ethanol, benzyl alcohol, propylene glycol, glycerin, etc.), and the like, and can contain pharmaceutical carriers such as stabilizers to prevent deterioration (e.g., ascorbic acid, sodium bisulfite, sodium pyrosulfite, BHA, tocopherol, EDTA, etc.), emulsifiers, buffers to regulate pH, and preservatives to inhibit microbial growth (e.g., phenylmercury nitrate, thimerosal, benzalkonium chloride, phenol, cresol, benzyl alcohol, etc.). The pharmaceutical composition can be administered in a pharmaceutically effective amount. At this time, the term "pharmaceutically effective amount" refers to an amount sufficient to exhibit an immuno-anticancer effect but not to cause side effects or a serious or excessive immune response. The exact dosage concentration depends on the composition to be administered and can be readily determined by those skilled in the art according to factors well known in the medical field, such as the patient's age, weight, health, gender, the patient's sensitivity to the drug, the route of administration, and the method of administration, and can be administered once or several times.

In another aspect of the present invention, the present invention provides a method of generating an immune response against cancer in a subject, comprising a step of administering the pharmaceutical composition comprising an adjuvant including lipopeptides and poly (I:C) as an active ingredient for the prevention or treatment of cancer to a subject except humans.

If the pharmaceutical composition is administered to a human (patient), it can be administered in an amount effective to stimulate an in vivo immune response, for example, it can be administered to a human once or several times, and the amount can be 0.25-3.6 mg, more preferably 0.45-1.8 mg, but not always limited thereto.

In another aspect of the present invention, the present invention provides a combination formulation comprising an adjuvant including lipopeptides and poly (I:C), and an immune checkpoint inhibitor as active ingredients for the prevention or treatment of cancer.

At this time, the immune checkpoint inhibitor can be at least one selected from the group consisting of an anti-CTLA4 antibody, an anti-PD-L1 antibody, and an anti-PD-1 antibody.

More specifically, the immune checkpoint inhibitor can be selected from the group consisting of an anti-CTLA4 antibody, a derivative thereof or an antigen-binding fragment thereof; an anti-PD-L1 antibody, a derivative thereof or an antigen-binding fragment thereof; an anti-LAG-3 antibody, a derivative thereof or an antigen-binding fragment thereof; an anti-OX40 antibody, a derivative thereof or an antigen-binding fragment thereof; an anti-TIM3 antibody, a derivative thereof or an antigen-binding fragment thereof; and an anti-PD-1 antibody, a derivative thereof or an antigen-binding fragment thereof. For example, as an immune checkpoint inhibitor targeting CTLA-4, Yervoy (Ipilimumab) can be used; as an immune checkpoint inhibitor targeting PD-L1, Tecentriq (Atezolizumab), Avelumab (Bavencio), Imfinzi (Durvalumab), and the like can be used alone or in combination; and as an immune checkpoint inhibitor targeting PD-1, Keytruda (Pembrolizumab), Opdivo (Nivolumab), Libtayo (Cemiplimab), and the like can be used alone or in combination.

In another aspect of the present invention, the present invention provides a combination formulation comprising an adjuvant including lipopeptides and poly (I:C), and a TLR (toll-like receptor) ligand as active ingredients for the prevention or treatment of cancer.

At this time, the TLR (Toll-like receptor) ligand can be one or more ligands selected from the group consisting of TLRs 1 to 13. For example, TLR7 ligand (Imiquimod, etc.), TLR7/8 ligands (Imidazoquinoline, etc.), and TLR9 ligand (CpG ODN, etc.) can be used.

In another aspect of the present invention, the present invention provides a combination formulation comprising an adjuvant including lipopeptides and poly (I:C), and saponin as active ingredients for the prevention or treatment of cancer.

At this time, the saponin can be selected from the group consisting of QS21, Quil A, QS7, QS17, and combinations thereof.

The immuno-oncology therapeutic composition comprising an adjuvant including lipopeptides and poly (I:C) as an active ingredient, provided in one aspect of the present invention, can induce a large therapeutic effect on a variety of carcinomas, and can be effectively used for anticancer therapy by significantly enhancing anticancer effects through combined administration with conventional anticancer drugs, such as chemical anticancer drugs, anticancer vaccines, and immune checkpoint inhibitors, having different mechanisms.

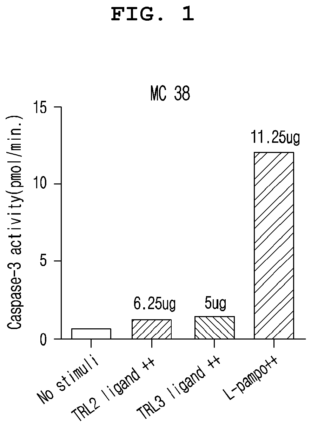

Specifically, it was confirmed that the immuno-oncology therapeutic composition comprising an adjuvant including lipopeptides and poly (I:C) of the present invention (hereinafter referred to as L-pampo) induced apoptosis of cancer cells by further activating capspase-3 of the caspase pathway in cancer cells more than when lipopeptide or poly(I:C) was treated alone (see Figure 1). Therefore, it was confirmed that L-pampo most strongly induced apoptosis of colon cancer cells.

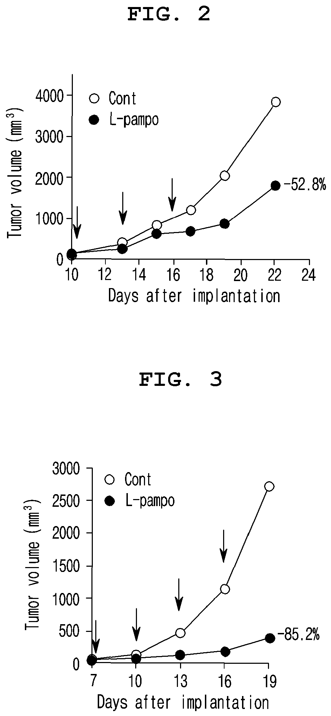

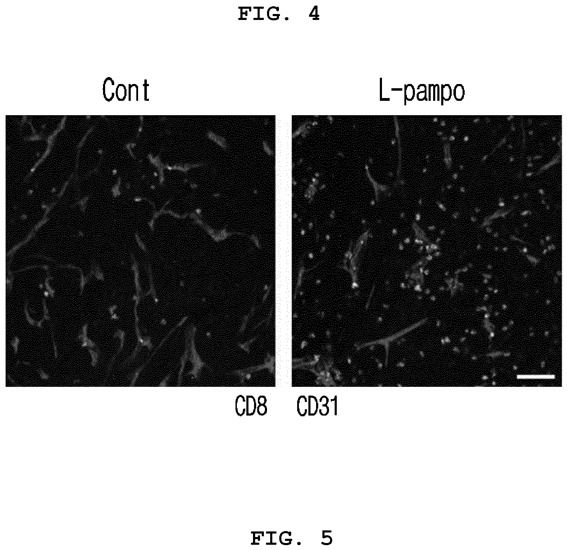

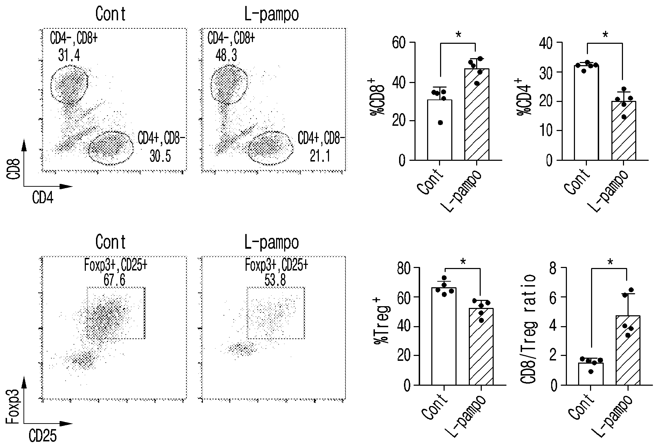

In addition, it was confirmed that L-pampo reduced tumor size in a melanoma mouse model (see Figure 2). In a colon cancer mouse model, L-pampo reduced tumor size, suggesting that it has anticancer efficacy (see Figure 3). Furthermore, it was confirmed that L-pampo of the present invention induced immune activity capable of killing tumors by inducing the infiltration of many CD8 T cells into the tumors of a colon cancer mouse model, while suppressing Treg+ cells (see Figures 4 and 5).

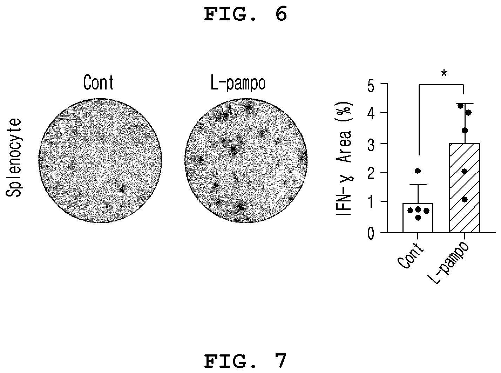

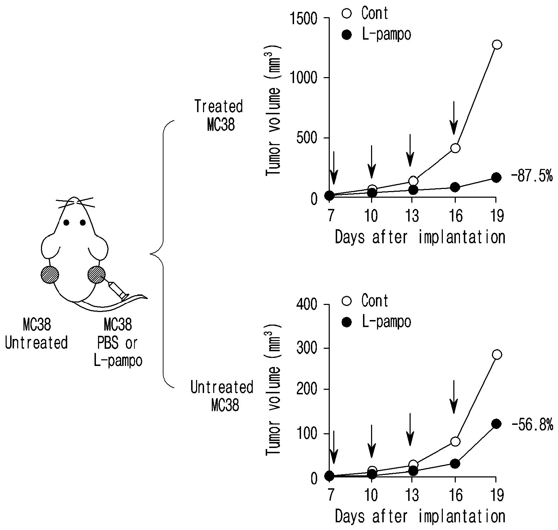

In addition, it was confirmed that L-pampo of the present invention strongly induced a tumor-specific cellular immune response by increasing the secretion of IFN-γ in a colon cancer mouse model (see Figure 6). It was also confirmed that L-pampo of the present invention showed abscopal efficacy, reducing the size of tumors treated with L-pampo as well as tumors not treated with L-pampo (see Figure 7).

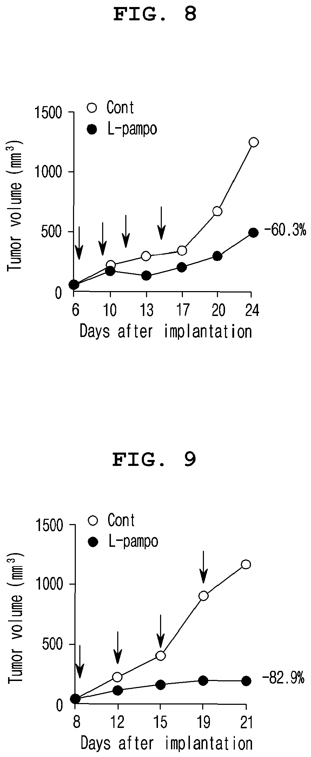

Meanwhile, it was confirmed that L-pampo reduced tumor size in a bladder cancer mouse model (see Figure 8). It was also confirmed that L-pampo reduced tumor size in a pancreatic cancer mouse model (see Figure 9). The above results suggest that L-pampo of the present invention exhibits anticancer activity in various cancers.

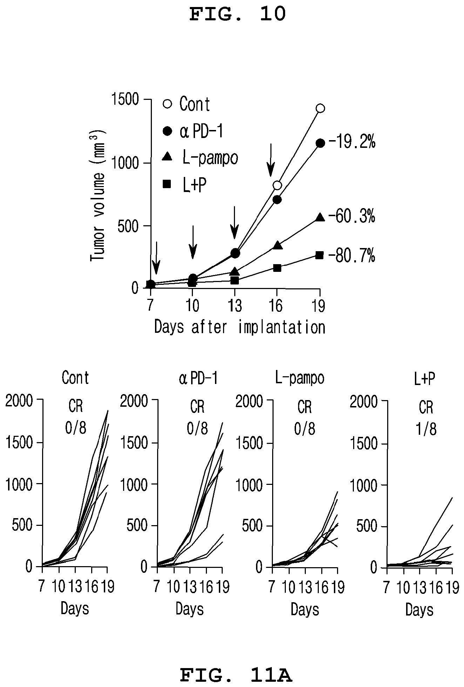

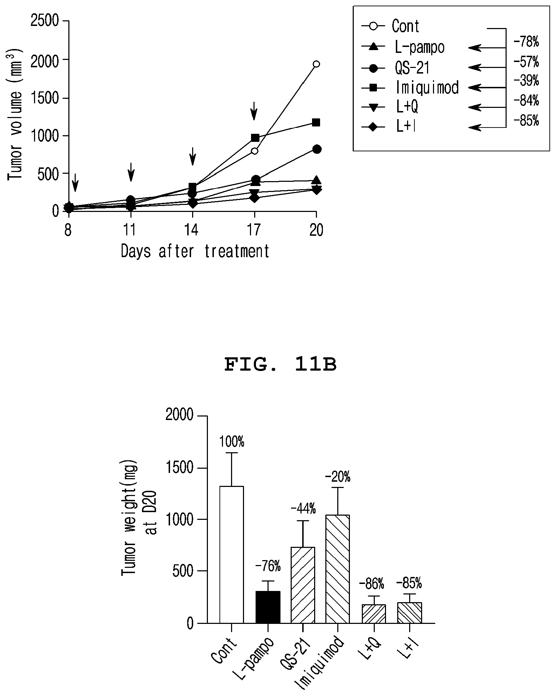

On the other hand, it was confirmed that when L-pampo of the present invention and PD-1 antibody, an immune checkpoint inhibitor, were administered in combination, the size of the tumor was significantly reduced compared to when administered alone (see Figure 10). Therefore, it was suggested that the combination treatment of L-pampo and immune checkpoint inhibitors exhibits strong anticancer efficacy. In addition, it was confirmed that when L-pampo of the present invention was administered in combination with another TLR ligand (Imiquimod) or saponin (QS-21), the size and weight of the tumor were significantly reduced compared to when administered alone (see Figures 11a and 11b). Therefore, it was confirmed that the combination treatment of the immuno-oncology therapeutic agent L-pampo of the present invention with other TLR ligands or saponin has a synergistic effect and can show strong anti-cancer efficacy.

The L-pampo comprising lipopeptides and poly(I:C) of the present invention induced a cancer-specific cellular immune response, infiltrated a large number of CD8+ T cells into the tumor, and showed strong anticancer efficacy that strongly inhibited tumor growth. From the above results, it was confirmed that the intratumoral administration of an immuno-oncology therapeutic composition containing L-pampo can be used as an immunotherapy agent that kills cancer cells and exhibits strong anticancer efficacy through cancer-specific immune responses and intratumoral infiltration of immune cells.

Hereinafter, the present invention will be described in detail by the following examples and experimental examples.

However, the following examples and experimental examples are only for illustrating the present invention, and the contents of the present invention are not limited thereto.

### Example 1: Analysis of caspase-3 activity of immuno-oncology drug comprising lipopeptide and poly(I:C) in MC38 cells, colon cancer cell line

### <1-1> Cell seeding and stimulation

MC38 cells, a colon cancer cell line, were seeded in a 12-well culture plate at the density of 1.5×10⁵ cells/well. The next day, the plate was treated with TLR2 ligand (Pam3CSK4), TLR3 ligand (Poly(I:C)), or L-pampo, a mixture of TLR2 (Pam3CSK4) and TLR3 (Poly(I:C)) ligands, and then cultured for 24 hours. At this time, the L-pampo was prepared by mixing 50 *µ*g of TLR2 ligand (Pam3CSK4) and 40 *µ*g of TLR3 ligand (Poly(I:C)).

### <1-2> Preparation of cell extract

Upon completion of 24 hours of the stimulation, cells were harvested, resuspended in approximately 100 µL of lysis buffer, and cultured on ice for 5 minutes. After centrifugation at 1000×g for 10 minutes at 4°C, the lysate of the supernatant was transferred to a new e-tube. After measuring the concentration of each sample through BCA analysis, the concentration of all samples was adjusted to be the same. These cell extract samples were stored at 4°C for immediate use and at -20°C for long-term storage.

### <1-3> Caspase-3 cell activity assay

The assay was performed according to the instructions of the caspase-3 cell activity assay kit (Millipore, #235419). Blank, negative control (cell extract treated with caspase-3 inhibitor), positive control (purified caspase-3), and cell extract of each sample were reacted with a caspase substrate (Ac-DEVD-pNA), and OD450 was measured through color development at intervals of 5 to 10 minutes for a minimum of 30 minutes to a maximum of 120 minutes. With a substrate standard, a conversion factor was calculated and the activity of each sample was converted and expressed as p mol/minute.

### <1-4> Caspase-3 activity comparative assay

The activity of caspase-3 was compared and analyzed in cell extract samples treated with negative control, positive control, TLR2 ligand, TLR3 ligand, and L-pampo.

The results are shown in Figure 1. Figure 1 is a graph confirming the caspase-3 activity of an immuno-oncology drug in a mouse colon cancer cell line.

As shown in Figure 1, it was confirmed that the caspase-3 activity was induced relatively higher in the experimental group treated with L-pampo compared to the experimental group treated with TLR2 ligand or TLR3 ligand. Therefore, it was confirmed that L-pampo most strongly induced apoptosis of MC38 cells, a colon cancer cell line.

### Example 2: Confirmation of efficacy of immuno-oncology drug comprising lipopeptide and poly(I:C) in melanoma mouse model

### <2-1> Construction of melanoma mouse model

Tumors were generated in mice by subcutaneous injection of 2×10⁵ B16F10 mouse melanoma cells into the right flank of 7-week-old female C57BL/6 mice (OrientBio, Korea).

### <2-2> Preparation and administration of immuno-oncology drug

The immuno-oncology drug L-pampo was prepared by mixing 50 *µ*g of Pam3CSK4 and 40 *µ*g of poly(I:C) . A buffer-administered group was set as a negative control group, and when the tumor size of the melanoma mouse model constructed in Example <2-1> above reached approximately 100 mm³, 90 *µ*g/dose of L-pampo was administered into the tumor three times at 3-day intervals.

### <2-3> Analysis of tumor suppressive efficacy

Tumor sizes for all experimental groups were measured every two to three days. The long and short axes of the tumor were measured, and the size of the tumor was calculated as [1/2 × size of the long axis × (size of the short axis)²]. The tumor suppressive efficacy of the immuno-oncology drug was analyzed based on the calculated tumor size.

The results are shown in Figure 2. Figure 2 is a graph confirming the tumor suppressive efficacy of an immuno-oncology drug in a melanoma mouse model.

As shown in Figure 2, in the experimental group administered with the immuno-oncology drug L-pampo, it was confirmed that the size of the tumor was reduced by about 52.8% on D22, 12 days after the start of administration, compared to the negative control group administered with buffer, showing that the immuno-oncology drug has melanoma inhibitory activity.

### Example 3: Confirmation of efficacy of immuno-oncology drug comprising lipopeptide and poly(I:C) in colon cancer mouse model

### <3-1> Construction of mouse model

Tumors were generated in mice by subcutaneous injection of 5×10⁴ MC38 mouse colon cancer cells into the right flank of 7-week-old female C57BL/6 mice (OrientBio, Korea).

### <3-2> Preparation and administration of immuno-oncology drug

The immuno-oncology drug L-pampo was prepared by mixing 100 *µ*g of Pam3CSK4 and 80 *µ*g of poly(I:C) . A buffer-administered group was set as a negative control group, and when the tumor size of the colon cancer mouse model constructed in Example <3-1> above reached approximately 50 mm³, 180 ug/dose of L-pampo was administered into the tumor four times at 3-day intervals.

### <3-3> Analysis of tumor suppressive efficacy

Tumor sizes for all experimental groups were measured every three days. The long and short axes of the tumor were measured, and the size of the tumor was calculated as [1/2 × size of the long axis × (size of the short axis)²]. The tumor suppressive efficacy of the immuno-oncology drug was analyzed based on the calculated tumor size.

The results are shown in Figure 3. Figure 3 is a graph confirming the tumor suppressive efficacy of an immuno-oncology drug in a colon cancer mouse model.

As shown in Figure 3, in the experimental group administered with the immuno-oncology drug L-pampo, it was confirmed that the size of the tumor was reduced by about 85.2% on D19, 12 days after the start of administration, compared to the negative control group administered with buffer, showing that the immuno-oncology drug has strong anticancer efficacy. This shows that the immuno-oncology drug L-pampo can strongly inhibit the growth of colon cancer.

### <3-4> Analysis of intratumoral immune cell infiltration

Tumors from all mice were collected on D19, 12 days after the start of administration, and intratumoral immune cell infiltration was analyzed using immunofluorescence staining and flow-cytometry analysis.

To perform immunofluorescence staining, the collected mouse tumors were fixed in 1% paraformaldehyde and then dehydrated using a 20% sucrose solution. The tumor tissues were frozen in OCT (optimal cutting temperature) compound, and the frozen tumor tissues were cut into 50 gm thick sections using a cryotome and fixed on slides. The tumor tissues fixed on the slides were reacted with a blocking solution for 1 hour. CD8 antibody and CD31 antibody were diluted 1:200 and reacted with the tumor tissues for 24 hours at 4°C. After the reaction, the tumor tissues were washed with PBST and then reacted with a fluorescent antibody and DAPI for 2 hours at room temperature. After mounting, fluorescence images were analyzed using a confocal microscope.

The results are shown in Figure 4.

Figure 4 is a set of photographs showing the results of analyzing the effect of an immuno-oncology drug on immune cell infiltration in the tumor using immunofluorescence staining in a colon cancer mouse model.

As shown in Figure 4, in the experimental group administered with L-pampo, significantly more CD8+ T cells (green fluorescence) were infiltrated into the tumor than in the experimental group administered with buffer, the negative control group. This shows that L-pampo can induce tumor suppression by immune cells by inducing the infiltration of many CD8 T cells into the tumor.

To perform flow cytometry, the collected mouse tumors were cut into small pieces, which were treated with collagenase D and DNase I, and reacted for 1 hour at 37°C. The reactant was filtered through a 70 *µ*m cell strainer, red blood cells were lysed, and then filtered again through a nylon mesh. The single cell suspension was blocked with CD16/32 antibodies and then stained with cell viable dye. After staining with fluorescent dye-conjugated CD45, CD3, CD8, CD4, Foxp3, and CD25, the reactant was measured using CytoFLEX flow cytometer. The measured results were quantitatively compared and analyzed using FlowJo software.

The results are shown in Figure 5.

Figure 5 is a set of graphs showing the results of analyzing the effect of an immuno-oncology drug on immune cell infiltration in the tumor using flow cytometry in a colon cancer mouse model.

As shown in Figure 5, in the experimental group administered with L-pampo, significantly more CD8+ T cells were infiltrated into the tumor than in the experimental group administered with buffer, the negative control group. In addition, it was confirmed that the experimental group administered with L-pampo had a significantly lower number of Treg+ cells, which suppress the activity of T cells in the tumor, compared to the negative control group. This shows that L-pampo can induce immune activity that can kill the tumor by infiltrating many CD8+ T cells into the tumor and suppressing Treg+ cells.

### <3-5> Analysis of tumor-specific cellular immune activity

On D19, 12 days after the start of administration, the spleens of all mice were harvested, and tumor-specific immune activity was analyzed through ELISPOT assay.

To perform tumor-specific ELISPOT assay, the collected spleens were ground through a cell strainer, spleen cells were separated, and dead cells were removed. Splenocytes and MC39 colon cancer cells were mixed in a 10:1 ratio and cultured together in a 96-well plate coated with mouse IFN-γ. The cells were cultured with a detection antibody for 2 hours at room temperature and then cultured with streptavidin-ALP for 1 hour at room temperature. After the culture, spots were confirmed by adding a substrate solution. Spots were analyzed using Image J software.

The results are shown in Figure 6.

Figure 6 is a set of photographs and a graph showing the results of analyzing the induction of tumor-specific cellular immune responses by an immuno-oncology drug using ELISPOT assay in a colon cancer mouse model.

As shown in Figure 6, in the experimental group administered with L-pampo, significantly more IFN-γ spot formation was induced compared to the experimental group administered with buffer, the negative control group. From the above results, it was confirmed that the intratumoral administration of the immuno-oncology drug L-pampo strongly induced a tumor-specific cellular immune response.

### <3-6> Analysis of abscopal efficacy in colon cancer mouse model

To establish an abscopal model, 5×10⁴ MC38 mouse colon cancer cells were injected subcutaneously into the right flank of 7-week-old female C57BL/6 mice (OrientBio, Korea), and 4 days later, 5×10⁴ MC38 colon cancer cells were injected subcutaneously into the left flank of the mice to generate tumors on both sides. The L-pampo prepared in Example <3-2> above was administered at 180 *µ*g/dose only to the tumor formed on the right side. The size of the tumors generated on both sides was measured at 3-day intervals to analyze the tumor suppressive efficacy.

The results are shown in Figure 7.

Figure 7 is a set of a diagram and graphs showing the results of analyzing the abscopal efficacy of an immuno-oncology drug in a colon cancer mouse model.

As shown in Figure 7, unlike the experimental group administered with buffer, the negative control group, the experimental group administered with L-pampo showed a significant decrease in the size of not only the tumor administered with the immuno-oncology drug L-pampo (right side) but also the tumor not administered (left side). From this, it was confirmed that when the immuno-oncology drug L-pampo was administered to the tumor, L-pampo not only killed cancer cells, but also killed the tumor not treated with L-pampo by inducing strong tumor-specific immune activity, indicating that the immuno-oncology drug L-pampo can also treat cancer cells that have metastasized.

### Example 4: Confirmation of efficacy of immuno-oncology drug comprising lipopeptide and poly(I:C) in bladder cancer mouse model

### <4-1> Construction of bladder cancer mouse model

Tumors were generated in mice by subcutaneous injection of 5×10⁵ MB49 mouse bladder cancer cells into the right flank of 7-week-old female C57BL/6 mice (OrientBio, Korea).

### <4-2> Preparation and administration of immuno-oncology drug

The immuno-oncology drug L-pampo was prepared by mixing 100 *µ*g of Pam3CSK4 and 80 *µ*g of poly(I:C). A buffer-administered group was set as a negative control group, and when the tumor size of the bladder cancer mouse model constructed in Example <4-1> above reached approximately 50 mm³, 180 ug/dose of L-pampo was administered into the tumor four times at 3-day intervals.

### <4-3> Analysis of tumor suppressive efficacy

Tumor sizes for all experimental groups were measured every three days. The long and short axes of the tumor were measured, and the size of the tumor was calculated as [1/2 × size of the long axis × (size of the short axis)²]. The tumor suppressive efficacy of the immuno-oncology drug was analyzed based on the calculated tumor size.

The results are shown in Figure 8. Figure 8 is a graph confirming the tumor suppressing efficacy of an immuno-oncology drug in a bladder cancer mouse model.

As shown in Figure 8, in the experimental group administered with the immuno-oncology drug L-pampo, it was confirmed that the size of the tumor was reduced by about 60.3% on D24, 18 days after the start of administration, compared to the negative control group administered with buffer, showing that the immuno-oncology drug has strong anticancer efficacy. This shows that the immuno-oncology drug L-pampo can strongly inhibit the growth of bladder cancer.

### Example 5: Confirmation of efficacy of immuno-oncology drug comprising lipopeptide and poly(I:C) in pancreatic cancer mouse model

### <5-1> Construction of pancreatic cancer mouse model

Tumors were generated in mice by subcutaneous injection of 5×10⁵ KPC mouse pancreatic cancer cells into the right flank of 8-week-old female C57BL/6 mice (OrientBio, Korea).

### <5-2> Preparation and administration of immuno-oncology drug

The immuno-oncology drug L-pampo was prepared by mixing 100 *µ*g of Pam3CSK4 and 80 *µ*g of poly(I:C) . A buffer-administered group was set as a negative control group, and when the tumor size of the pancreatic cancer mouse model constructed in Example <5-1> above reached approximately 50 mm³, 180 ug/dose of L-pampo was administered into the tumor four times at 3-day intervals.

### <5-3> Analysis of tumor suppressive efficacy

Tumor sizes for all experimental groups were measured every three days. The long and short axes of the tumor were measured, and the size of the tumor was calculated as [1/2 × size of the long axis × (size of the short axis)²]. The tumor suppressive efficacy of the immuno-oncology drug was analyzed based on the calculated tumor size.

The results are shown in Figure 9.

Figure 9 is a graph confirming the tumor suppressing efficacy of an immuno-oncology drug in a pancreatic cancer mouse model.

As shown in Figure 9, in the experimental group administered with the immuno-oncology drug L-pampo, it was confirmed that the size of the tumor was reduced by about 82.9% on D21, 13 days after the start of administration, compared to the negative control group administered with buffer, showing that the immuno-oncology drug has strong anticancer efficacy. This shows that the immuno-oncology drug L-pampo can strongly inhibit the growth of pancreatic cancer.

### Example 6: Confirmation of efficacy of immuno-oncology drug comprising lipopeptide and poly(I:C) in combination with immune checkpoint inhibitor in colon cancer mouse model

### <6-1> Construction of colon cancer mouse model

Tumors were generated in mice by subcutaneous injection of 1×10⁵ MC38 mouse colon cancer cells into the right flank of 8-week-old female C57BL/6 mice (OrientBio, Korea).

### <6-2> Preparation and administration of immuno-oncology drug L-pampo

The immuno-oncology drug L-pampo was prepared by mixing 27.8 *µ*g of Pam3CSK4 and 22.2 *µ*g of poly(I:C). A buffer-administered group was set as a negative control group, and when the tumor size of the colon cancer mouse model constructed in Example <6-1> above reached approximately 50 mm³, 50 ug/dose of L-pampo was administered into the tumor four times at 3-day intervals.

### <6-3> Administration of immune checkpoint inhibitor

A mouse PD-1 antibody, which is an immune checkpoint inhibitor, was purchased from BioXCell. A group administered with the mouse PD-1 antibody alone was set as a combination control group. The combined administration group was treated with 200 *µ*g/dose of mouse PD-1 antibody was intraperitoneally administered 4 times at 3-day intervals during intratumoral administration of L-pampo.

### <6-4> Analysis of tumor suppressive efficacy

Tumor sizes for all experimental groups were measured every three days. The long and short axes of the tumor were measured, and the size of the tumor was calculated as [1/2 × size of the long axis × (size of the short axis)²]. The tumor suppressive efficacy was analyzed based on the calculated tumor size.

The results are shown in Figure 10.

Figure 10 is a set of graphs confirming the efficacy of an immuno-oncology drug in combination with an immune checkpoint inhibitor in a colon cancer mouse model.

As shown in Figure 10, in the experimental group administered with the PD-1 antibody, it was confirmed that the size of the tumor was reduced by about 19.2% on D19, 12 days after the start of administration, compared to the negative control group administered with buffer. On the other hand, in the experimental group administered with the immuno-oncology drug L-pampo, it was confirmed that the size of the tumor was reduced by about 60.3% on D19, indicating the immuno-oncology drug L-pampo showed a stronger tumor suppressive effect than the immune checkpoint inhibitor PD-1 antibody. In addition, in the experimental group administered with the immuno-oncology drug L-pampo and the immune checkpoint inhibitor PD-1 antibody together, it was confirmed that the size of the tumor was reduced by about80.7% on D19, and in one of eight animals, the tumor was completely disappeared. From the above results, it was confirmed that the combination treatment with the immuno-oncology drug L-pampo and the immune checkpoint inhibitor can show strong anticancer efficacy.

### Example 7: Confirmation of efficacy of immuno-oncology drug comprising lipopeptide and poly(I:C) in combination with other TLR ligands or saponin in colon cancer mouse model

### <7-1> Construction of colon cancer mouse model

Tumors were generated in mice by subcutaneous injection of 1×10⁵ MC38 mouse colon cancer cells into the right flank of 8-week-old female C57BL/6 mice (OrientBio, Korea).

### <7-2> Preparation and administration of immuno-oncology drug L-pampo

The immuno-oncology drug L-pampo was prepared by mixing 25 *µ*g of Pam3CSK4 and 20 *µ*g of poly(I:C) . A buffer-administered group was set as a negative control group, and when the tumor size of the colon cancer mouse model constructed in Example <7-1> above reached approximately 50 mm³, 45 *µ*g/dose of L-pampo was administered into the tumor four times at 3-day intervals.

### <7-3> Preparation and administration of co-administered substance of immuno-oncology drug L-pampo

A co-administered substance was prepared by vortex-mixing 45 *µ*g/dose of the immuno-oncology drug L-pampo prepared in Example <7-2> above with 10 *µ*g/dose of QS21 (Creative biolabs) or 10 *µ*g/dose of Imiquimod (Invivogen), respectively. This was administered into the tumor four times at 3-day intervals.

### <7-4> Analysis of tumor suppressive efficacy

Tumor sizes for all experimental groups were measured every three days. The long and short axes of the tumor were measured, and the size of the tumor was calculated as [1/2 × size of the long axis × (size of the short axis)²]. The tumor suppressive efficacy was analyzed based on the calculated tumor size.

The results are shown in Figure 11.

Figure 11 is a set of graphs confirming the efficacy of an immuno-oncology drug in combination with other TLR ligands or saponin in a colon cancer mouse model.

As shown in Figure 11a, in the experimental group administered with imiquimod (TLR 7 ligand), another TLR ligand, it was confirmed that the size of the tumor was reduced by about 39% on D20, 12 days after the start of administration, compared to the negative control group administered with buffer. In addition, in the experimental group administered with the saponin QS-21, it was confirmed that the size of the tumor was reduced by about 57% on D20. On the other hand, in the experimental group administered with L-pampo, it was confirmed that the size of the tumor was reduced by about 78% on D20, showing the strongest tumor growth inhibition effect among the groups administered alone. It was also confirmed that the tumor size was reduced by more than 84% on D20 in all experimental groups administered with the immuno-oncology drug L-pampo in combination with imiquimod or QS-21, respectively. As shown in Figure 11b, a similar trend of tumor size suppression was confirmed in the results of measuring the tumor weight of each experimental group on D20. Therefore, it was confirmed that the combination administration of the immuno-oncology drug L-pampo with other TLR ligands or saponin can show a synergistic effect and show strong anticancer efficacy.

## Claims

1. A pharmaceutical composition for use in preventing or treating cancer comprising an adjuvant including lipopeptides and poly (I:C) as an active ingredient.

2. The pharmaceutical composition according to claim 1, wherein the pharmaceutical composition prevents or treats cancer through activating a T cell immune response.

3. The pharmaceutical composition according to claim 1, wherein the lipopeptide is at least one selected from the group consisting of Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK and Dhc-SKKKK.

4. The pharmaceutical composition according to claim 1, wherein the lipopeptide and poly(I:C) are included at a weight ratio of 0.1 to 10:1.

5. The pharmaceutical composition according to claim 1, wherein the pharmaceutical composition is an aqueous solution formulation.

6. A method of generating an immune response against cancer in a subject, comprising a step of administering the pharmaceutical composition comprising an adjuvant including lipopeptides and poly (I:C) as an active ingredient for the prevention or treatment of cancer to a subject except humans.

7. A pharmaceutical combination formulation for use in preventing or treating cancer comprising an adjuvant including lipopeptides and poly (I:C), and an immune checkpoint inhibitor as active ingredients.

8. The pharmaceutical combination formulation according to claim 7, wherein the immune checkpoint inhibitor is at least one selected from the group consisting of an anti-CTLA4 antibody, an anti-PD-L1 antibody, and an anti-PD-1 antibody.

9. A combination formulation for use in preventing or treating cancer comprising an adjuvant including lipopeptides and poly (I:C), and a TLR (toll-like receptor) ligand as active ingredients.

10. The combination formulation according to claim 9, wherein the TLR (toll-like receptor) ligand is one or more ligands selected from the group consisting of TLRs 1 to 13.

11. A combination formulation for use in preventing or treating cancer comprising an adjuvant including lipopeptides and poly (I:C), and saponin as active ingredients.

12. The combination formulation according to claim 11, wherein the saponin is selected from the group consisting of QS21, Quil A, QS7, QS17, and combinations thereof.

13. A method for preventing or treating cancer, comprising a step of administering a lipopeptide and poly(I:C) to a subject.

14. A method for preventing or treating cancer, comprising a step of administering a lipopeptide, poly(I:C) and an immune checkpoint inhibitor to a subject.

15. A method for preventing or treating cancer, comprising a step of administering a lipopeptide, poly(I:C) and a TLR (toll-like receptor) ligand to a subject.

16. A method for preventing or treating cancer, comprising a step of administering lipopeptide, poly(I:C) and saponin to a subject.

17. A use of lipopeptides and poly(I:C) for the preparation of a medicament for the prevention, amelioration or treatment of cancer.

18. A use of lipopeptides, poly(I:C), and immune checkpoint inhibitors for the preparation of a medicament for the prevention, amelioration or treatment of cancer.

19. A use of lipopeptides, poly(I:C), and TLR (toll-like receptor) ligands for the preparation of a medicament for the prevention, amelioration, or treatment of cancer.

20. A use of lipopeptides, poly(I:C), and saponin for the preparation of a medicament for the prevention, amelioration, or treatment of cancer.
